# EUROPÄISCHE PATENTSCHRIFT

(11) **EP 1 773 760 B1**
(45) Veröffentlichungstag und Bekanntmachung des Hinweises auf die Patenterteilung: **11.01.2017**
(21) Anmeldenummer: 05774614.1
(22) Anmeldetag: 13.07.2005
(51) Int. Cl.: C08F 8/30, C12P 17/10, C12P 13/02, C07D 231/08

(54) **KATALYSIERTES VERFAHREN ZUR HERSTELLUNG VON (METH)ACRYLATEN VON N-HYDROXYALKYLIERTEN AMIDEN**
CATALYTIC METHODS FOR THE PRODUCTION OF (METH)ACRYLATES FROM N-HYDROXYALKYLATED AMIDES
PROCEDE DE PRODUCTION CATALYTIQUE DE (METH)ACRYLATES D'AMIDES N-HYDROXYALKYLES

(30) Priorität: 29.07.2004 DE 102004036930; 11.05.2005 US 679665 P
(43) Veröffentlichungstag der Anmeldung: 18.04.2007
(73) Patentinhaber: BASF SE, 67056 Ludwigshafen am Rhein (DE)
(72) Erfinder: HÖFER, Frank, 67098 Bad Dürkheim (DE); HÄRING, Dietmar, 69198 Schriesheim (DE)
(74) Vertreter: BASF IP Association
(86) Internationale Anmeldenummer: PCT/EP2005/007576
(87) Internationale Veröffentlichungsnummer: WO 2006/012980

(56) Entgegenhaltungen:
- EP-A- 0 236 994
- EP-A- 0 263 749
- EP-A1- 0 263 749
- WO-A-2004/050888
- US-A- 2 871 223
- PROBST J ET AL: "HOMO- UND COPOLYMERISATION VON N,N-DISUBSTITUIERTEN CARBAMOYLOXYALKYLACRYLATEN UND -METHACRYLATEN" MAKROMOLEKULARE CHEMIE, MACROMOLECULAR CHEMISTRY AND PHYSICS, HUTHIG UND WEPF VERLAG, BASEL, CH, Bd. 177, 1976, Seiten 2681-2695, XP009026952 ISSN: 0025-116X
- DERANGO R ET AL: "THE LIPASE-CATALYZED SYNTHESIS OF CARBAMOYLOXYETHYL METHACRYLATE", BIOTECHNOLOGY LETTERS, SPRINGER NETHERLANDS, NL, vol. 16, no. 3, 1 March 1994 (1994-03-01), pages 241-246, XP009026932, ISSN: 0141-5492, DOI: 10.1007/BF00134619

## Beschreibung

Die vorliegende Erfindung betrifft ein Verfahren zur katalytischen Herstellung von (Meth)acrylaten von N-hydroxyalkylierten Amiden und deren Verwendung.

Die Herstellung von (Meth)acrylsäureestern erfolgt zumeist durch katalytische Veresterung von (Meth)acrylsäure oder Umesterung von anderen (Meth)acrylsäureestern mit Alkoholen in Gegenwart starker Säuren oder starker Basen. Säure- oder baseempfindliche (Meth)acrylsäureester lassen sich daher oftmals durch eine Ver- oder Umesterung in der Regel nicht gezielt herstellen.

Ureidoalkohole im Sinne der vorliegenden Schrift sind solche Verbindungen, die mindestens eine N-(C=O)-N-Gruppe und mindestens eine Hydroxygruppe (-OH) aufweisen. Analog sind N-hydroxyalkylierte Amide im Sinne der vorliegenden Schrift solche Verbindungen, die mindestens eine Z1-(C=O)-N-Gruppe und mindestens eine Hydroxygruppe (-OH) aufweisen, wobei die Hydroxygruppe mit dem Stickstoffatom der Z¹-(C=O)-N-Gruppe verbunden ist und Z¹ definiert ist wie unten aufgeführt.

Derartige Ureidoalkohole und N-Hydroxyalkylierte Amide hydrolysieren unter Säure-oder Baseeinfluß äußerst leicht. Zudem ist besonders bei der Reaktion mit Base in Gegenwart von (Meth)acrylat mit Nebenreaktionen beispielsweise im Sinne einer Michael-Addition zu rechnen, siehe z.B. EP-A1 236 994, S. 3, Z. 47 ff.

EP-A1 236 994 beschreibt die Herstellung von Ureido(meth)acrylaten durch Umesterung von (Meth)acrylat in Gegenwart von Titanalkoholaten oder 1,3-Dicarbonylchelaten von Titan, Zirkonium, Eisen oder Zink.

Nachteilig an diesen Verbindungen ist, daß die Metallverbindungen feuchtigkeitsempfindlich sind und daher leicht desaktivieren. Zudem beinflussen im Produkt verbleibende Spuren der Katalysatoren eine eventuell nachfolgende Polymerisation, und müssen daher aufwendig aus dem Produkt entfernt werden. Eine solche Entfernung wird zumeist mittels einer wäßrigen Wäsche durchgeführt, so daß das Produkt anschließend getrocknet werden muß.

JP-A 62-185 059 und JP-A 62-175 448 beschreiben die Umsetzung von (Meth)acrylaten mit Alkylaminoalkoholen in Gegenwart von Alkalimetallcarbonaten. JP-A 62-230 755 beschreibt die Umsetzung von (Meth)acrylaten mit Alkylaminoalkoholen in Gegenwart von Alkalimetallphosphaten.

Die Offenbarung ist auf Alkyl- und Dialkylaminoalkohole beschränkt und enthält keinen Hinweis auf Ureidoalkohole oder N-hydroxyalkylierte Amide.

Aus US 2,871,223 ist die Herstellung von Ureido(meth)acrylaten durch Reaktion von Ureidoalkoholen mit (Meth)Acrylsäurechlorid bekannt.

Die Verwendung von (Meth)Acrylsäurechlorid bei den beschriebenen Umsetzungen führt zur Salzbildung und wegen dessen hoher Reaktivität zu unselektiven Reaktionen, wie beispielsweise Diacrylierungen.

Die Herstellung von (Meth)acrylsäureestern durch eine enzymatische Ver- oder Umesterung ist generell bekannt, beispielsweise aus EP-A1 999 229.

Derango et al. beschrieben in Biotechnol. Lett. 1994, 16, 241-246 die Lipase-katalysierte Herstellung von Carbamoyloxyethylmethacrylat durch Umesterung von 2-Hydroxyethylcarbamat mit Vinylmethacrylat. Eine vollständige Umsetzung wird erreicht durch das spezielle Edukt Vinylmethacrylat, da freigesetzter Vinylalkohol dem Reaktionsgleichgewicht als Acetaldehyd entzogen wird. Nachteilig an diesem Verfahren ist, daß Vinylmethacrylat nicht kommerziell verfügbar ist. Zudem handelt es sich dabei um O hydroxyalkylierte Carbamoyle und stellen somit ein anderes Reaktionssystem dar als die hier behandelten Ureidoalkohole und N-hydroxyalkylierten Amide.

In WO 2004/50888 werden weitere O-hydroxyalkylierte Carbamoyle enzymatisch mit (Meth)Acrylsäure(ester) ver- bzw. umgeestert. Auch hier handelt es sich um ein anderes Reaktionssystem als die hier behandelten Ureidoalkohole und N-hydroxyalkylierte Amide.

Aufgabe der vorliegenden Erfindung war es, ein weiteres Verfahren zur Verfügung zu stellen, mit dem (Meth)acrylate von N-hydroxyalkylierten Amiden in hohen Umsätzen und hohen Reinheiten aus einfachen Edukten herstellbar sind. Die Synthese sollte unter milden Bedingungen ablaufen, so daß Produkte mit einer niedrigen Farbzahl und hoher Reinheit resultieren. Insbesondere soll der Anteil von mehrfach (meth)acrylierten Produkten zurückgedrängt werden. Ein notwendiger Katalysator soll ferner einfach abtrennbar sein und keine weiteren Nachbehandlungen, z.B. in Form von Aufarbeitungsschritten, des Reaktionsproduktes zur Folge haben.

Die Aufgabe wurde gelöst durch ein Verfahren zur Herstellung von (Meth)acrylaten von N-hydroxyalkylierten Amiden in dem man ringförmige N-hydroxyalkylierte Amide (C), 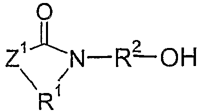 oder offenkettige N-hydroxyalkylierte Amide (O) 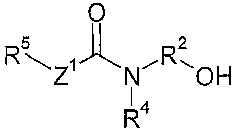 worin
Z¹ Sauerstoff, Schwefel, unsubstituierter oder substituierter Phosphor oder un- oder monosubstituierter Stickstoff (N-R³),
R¹ und R² jeweils unabhängig voneinander C₂-C₂₀-Alkylen, C₅-C₁₂-Cycloakylen, C₆-C₁₂-Arylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, oder
R²-OH für eine Gruppe der Formel -[Xᵢ]ₖ-H,
R³, R⁴ und R⁵ unabhängig voneinander jeweils Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, wobei R³ und R⁵ auch gemeinsam einen fünf- bis zwölfgliedrigen Ring bilden können,
mit der Maßgabe, daß im Fall von Z¹ = O R⁵ lediglich unsubstituiertes C₁ - C₁₈-Alkyl, C₆ - C₁₂-Aryl oder C₅ - C₁₂-Cycloalkyl ist,
k für eine Zahl von 1 bis 50 und
Xᵢ für jedes i = 1 bis k unabhängig voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O- und -CHPh-CH₂-O-, worin Ph für Phenyl und Vin für Vinyl steht,
bedeuten,

in Gegenwart mindestens eines heterogenen Katalysators ausgewählt aus der Gruppe bestehend aus anorganischen Phosphat-Salzen (S) , wobei das mindestens eine anorganische Phosphat-Salz (S) einen pKB -Wert von nicht mehr als 7,0 und nicht weniger als 1,0 sowie einen Löslichkeit im Reaktionsmedium bei 25 °C von nicht mehr als 1 g/I aufweist, und Enzymen (E), wobei das Enzym ausgewählt ist aus der Gruppe bestehend aus Lipase aus Candida antarctica B, Lipase aus Alcaligenes sp., Aspergillus sp., Mucor sp., Penicilium sp., Geotricum sp., Rhizopus sp., Burkholderia sp., Candida sp., Pseudomonas sp., Thermomyces sp. oder Schweinepankreas, mit (Meth)acrylsäure verestert oder mindestens einem (Meth)acrylsäureester (D) umestert.

Mit Hilfe des erfindungsgemäßen Verfahrens ist die Herstellung von (Meth)acrylaten von N-hydroxyalkylierten Amiden in hoher Ausbeute, unter milden Bedingungen, mit guten Farbzahlen und ohne Waschschritte zur Aufreinigung von Produkten möglich (Meth)acrylsäure steht in dieser Schrift für Methacrylsäure und Acrylsäure, bevorzugt für Methacrylsäure.

In den obigen Definitionen bedeuten
gegebenenfalls durch Aryl, Alkyl, Aryloxy, Alkyloxy, Heteroatome und/oder Heterocyclen substituiertes C₂-C₂₀-Alkylen beispielsweise 1,2-Ethylen, 1,2-Propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen, 2,2-Dimethyl-1,4-butylen,
gegebenenfalls durch Aryl, Alkyl, Aryloxy, Alkyloxy, Heteroatome und/oder Heterocyclen substituiertes C₅-C₁₂-Cycloalkylen beispielsweise Cyclopropylen, Cyclopentylen, Cyclohexylen, Cyclooctylen, Cyclododecylen,
gegebenenfalls durch Aryl, Alkyl, Aryloxy, Alkyloxy, Heteroatome und/oder Heterocyclen substituiertes, durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen beispielsweise 1-Oxa-1,3-propylen, 1,4-Dioxa-1,6-hexylen, 1,4,7-Trioxa-1,9-nonylen, 1-Oxa-1,4-butylen, 1,5-Dioxa-1,8-octylen, 1-Oxa-1,5-pentylen, 1-Oxa-1,7-heptylen, 1,6-Dioxa-1,10-decylen, 1-Oxa-3-methyl-1,3-propylen, 1-Oxa-3-methyl-1,4-butylen, 1-Oxa-3,3-dimethyl-1,4-butylen, 1-Oxa-3,3-dimethyl-1,5-pentylen, 1,4-Dioxa-3,6-dimethyl-1,6-hexylen, 1-Oxa-2-methyl-1,3-propylen, 1,4-Dioxa-2,5-dimethyl-1,6-hexylen, 1-Oxa-1,5-pent-3-enylen, 1-Oxa-1,5-pent-3-inylen, 1,1-, 1,2-, 1,3- oder 1,4-Cyclohexylen, 1,2- oder 1,3-Cyclopentylen, 1,2-, 1,3- oder 1,4-Phenylen, 4,4'-Biphenylen, 1,4-Diaza-1,4-butylen, 1-Aza-1,3-propylen, 1,4,7-Triaza-1,7-heptylen, 1,4-Diaza-1,6-hexylen, 1,4-Diaza-7-oxa-1,7-heptylen, 4,7-Diaza-1-oxa-1,7-heptylen, 4-Aza-1-oxa-1,6-hexylen, 1-Aza-4-oxa-1,4-butylen, 1-Aza-1,3-propylen, 4-Aza-1-oxa-1,4-butylen, 4-Aza-1,7-dioxa-1,7-heptylen, 4-Aza-1-oxa-4-methyl-1,6-hexylen, 4-Aza-1,7-dioxa-4-methyl-1,7-heptylen, 4-Aza-1,7-dioxa-4-(2'-hydroxyethyl)-1,7-heptylen, 4-Aza-1-oxa-(2'-hydroxyethyl)-1,6-hexylen oder 1,4-Piperazinylen,
gegebenenfalls durch Aryl, Alkyl, Aryloxy, Alkyloxy, Heteroatome und/oder Heterocyclen substituiertes C₆-C₁₂-Arylen beispielsweise 1,2-, 1,3- oder 1,4-Phenylen, 4,4'-Biphenylen, Toluylen oder Xylylen,
C₁-C₁₈-Alkyl oder gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hexadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonylethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl,
gegebenenfalls durch ein oder mehrere Sauerstoff- und/oder Schwefelatome und/oder ein oder mehrere substituierte oder unsubstituierte Iminogruppen unterbrochenes C₂-C₁₈-Alkenyl beispielsweise Vinyl, 1-Propenyl, Allyl, Methallyl, 1,1-Dimethylallyl, 2-Butenyl, 2-Hexenyl, Octenyl, Undecenyl, Dodecenyl, Octadecenyl, 2-Phenylvinyl, 2-Methoxyvinyl, 2-Ethoxyvinyl, 2-Methoxyallyl, 3-Methoxyallyl, 2-Ethoxyallyl, 3-Ethoxyallyl oder 1- oder 2-Chlorvinyl,
gegebenenfalls durch ein oder mehrere Sauerstoff- und/oder Schwefelatome und/oder ein oder mehrere substituierte oder unsubstituierte Iminogruppen unterbrochenes C₆-C₁₂-Aryl beispielsweise Phenyl, Tolyl, Xylyl, α-Naphthyl, β-Naphthyl, 4-Diphenyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch ein oder mehrere Sauerstoff- und/oder Schwefelatome und/oder ein oder mehrere substituierte oder unsubstituierte Iminogruppen unterbrochenes C₅-C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl,
und
gegebenenfalls durch ein oder mehrere Sauerstoff- und/oder Schwefelatome und/oder ein oder mehrere substituierte oder unsubstituierte Iminogruppen unterbrochenes fünfbis sechsgliedrigen, Sauerstoff-, Stickstoff- und/oder Schwefelatome aufweisenden Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl.

Beispiele für R¹ sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl-1,4-butylen, 1,2-Cyclopentylen, 1,3-Cyclopentylen, 1,2-Cyclohexylen,1,3-Cyclohexylen und ortho-Phenylen
bevorzugt sind 1,2-Ethylen, 1,2-Propylen, 1,3-Propylen,
besonders bevorzugt sind 1,2-Ethylen und 1,2-Propylen und
ganz besonders bevorzugt ist 1,2-Ethylen.

Beispiele für R² sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl-1,4-butylen, 1,2-Cyclopentylen, 1,3-Cyclopentylen, 1,2-Cyclohexylen,1,3-Cyclohexylen und ortho-Phenylen
bevorzugt sind 1,2-Ethylen, 1,2-Propylen, 1,3-Propylen,
besonders bevorzugt sind 1,2-Ethylen und 1,2-Propylen und
ganz besonders bevorzugt ist 1,2-Ethylen.

Beispiele für R³ und R⁵ sind unabhängig voneinander Wasserstoff oder C₁-C₄-Alkyl, was im Rahmen dieser Schrift für Methyl, Ethyl, *iso*-Propyl, n-Propyl, n-Butyl, *iso*-Butyl, *sek*-Butyl oder *tert*-Butyl steht, bevorzugt für Wasserstoff, Methyl, Ethyl, n-Propyl und n-Butyl, besonders bevorzugt für Wasserstoff, Methyl, Ethyl und n-Butyl und ganz besonders bevorzugt für Wasserstoff, Methyl und Ethyl und insbesondere für Wasserstoff.

Wenn R³ und R⁵ einen gemeinsamen Ring bilden, so können R³ und R⁵ gemeinsam 1,4-Butylen, 1,5-Pentylen oder 3-Oxa-1,5-pentylen bedeuten.

Beispiele für R⁴ sind Wasserstoff, Methyl, Ethyl, *iso*-Propyl, n-Propyl, n-Butyl, *iso*-Butyl, *sek*-Butyl, *tert*-Butyl, n-Hexyl, n-Heptyl, n-Octyl, n-Decyl, n-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, Phenyl, Naphthyl oder Benzyl.

Z¹ ist bevorzugt O oder NR³, besonders bevorzugt NR³.

Bevorzugt sind die ringförmigen Alkohole (C) gegenüber den offenkettigen Alkoholen (O).

Bevorzugte Individuen (C) sind 2'-Hydroxyethyl-ethylenharnstoff, 3'-Hydroxypropyl-ethylenharnstoff, 2'-Hydroxypropyl-ethylenharnstoff, 2'-Hydroxyethyl-1,3-propylenharnstoff, 2'-Hydroxypropyl-1,3-propylenharnstoff, 3'-Hydroxypropyl-1,3-propylenharnstoff, 2'-Hydroxyethyl-1,2-propylenharnstoff, 2'-Hydroxypropyl-1,2-propylenharnstoff, 3'-Hydroxypropyl-1,2-propylenharnstoff, 1-(2'-Hydroxy-ethyl)-imidazolidin-2,4-dion, 1-(2'-Hydroxy-ethyl)-dihydro-pyrimidin-2,4-dion oder 3-(2-Hydroxy-ethyl)-oxazolidin-2-on. Besonders bevorzugt sind 2'-Hydroxyethyl-ethylenharnstoff, 3'-Hydroxypropyl-ethylenharnstoff, 2'-Hydroxyethyl-1,3-propylenharnstoff und 3'-Hydroxypropyl-1,3-propylenharnstoff.

Bevorzugte Individuen (O) sind N-(2-Hydroxyethyl)harnstoff, N-(2-Hydroxypropyl)harnstoff, N-(3-Hydroxypropyl)harnstoff, N',N'-Dimethyl-N-(2-Hydroxyethyl)harnstoff, N',N'-Dimethyl-N-(2-Hydroxypropyl)harnstoff, N',N'-Dimethyl-N-(3-Hydroxypropyl)harnstoff, N',N'-Diethyl-N-(2-Hydroxyethyl)harnstoff, N',N'-Diethyl-N-(2-Hydroxypropyl)harnstoff, N',N'-Diethyl-N-(3-Hydroxypropyl)harnstoff, N',N'-Di-n-butyl-N-(2-Hydroxyethyl)harnstoff, N',N'-Di-n-butyl-N-(2-Hydroxypropyl)harnstoff und N',N'-Di-n-butyl-N-(3-Hydroxypropyl)harnstoff, besonders bevorzugt sind N-(2-Hydroxyethyl)harnstoff, N-(2-Hydroxypropyl)harnstoff und N-(3-Hydroxypropyl)harnstoff.

In Schritt c) erfolgt die Veresterung mit (Meth)acrylsäure oder bevorzugt die Umesterung des Alkohols (C) oder (O) mit mindestens einem, bevorzugt einem (Meth)acrylat (D) in Anwesenheit mindestens eines heterogenen Katalysators, ausgewählt aus der Gruppe bestehend aus anorganischen Phosphat-Salzen (S) und Lipasen (E) ausgewählt aus der Gruppe bestehend aus Candida antarctica B, Lipase aus Alcaligenes sp., Aspergillus sp., Mucor sp., Penicilium sp., Geotricum sp., Rhizopus sp., Burkholderia sp., Candida sp., Pseudomonas sp., Thermomyces sp. oder Schweinepankreas.

Verbindungen (D) können (Meth)acrylsäure oder Ester von (Meth)acrylsäure mit einem gesättigten Alkohol sein, bevorzugt gesättigte C₁ - C₁₀-Alkylester der (Meth)acrylsäure, besonders bevorzugt gesättigte C₁ - C₄-Alkylester der (Meth)acrylsäure.

Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für Verbindungen (D) sind (Meth)acrylsäuremethyl-, -ethyl-, -n-butyl-, *-iso-*butyl-, -*tert*-butyl-, n-octyl- und -2-Ethylhexylester, 1,2-Ethylenglycoldi- und-mono(meth)acrylat, 1,4-Butandioldi- und -mono(meth)acrylat, 1,6-Hexandioldi- und-mono(meth)acrylat, Trimethylolpropantri(meth)acrylat und Pentaerythrit-tetra(meth)acrylat.

Besonders bevorzugt sind (Meth)acrylsäuremethyl-, -ethyl-, -n-butyl- und -2-Ethylhexylester, ganz besonders bevorzugt (Meth)acrylsäuremethyl-, -ethyl- und -n-butylester, insbesondere (Meth)acrylsäuremethyl- und -ethylester und speziell (Meth)acrylsäuremethylester.

Erfindungsgemäß einsetzbare anorganischen Salzen (S) sind solche, die einen pK_{B}-Wert von nicht mehr als 7,0, bevorzugt von nicht mehr als 6,1 und besonders bevorzugt von nicht mehr als 4,0 aufweisen. Gleichzeitig sollte der pK_{B}-Wert 1,0 nicht unterschreiten, bevorzugt nicht weniger als 1,5 betragen und besonders bevorzugt nicht weniger als 1,6.

Heterogene Katalysatoren sind im Rahmen dieser Schrift erfindungsgemäß solche, die eine Löslichkeit im Reaktionsmedium bei 25 °C von nicht mehr als 1 g/l aufweisen, bevorzugt von nicht mehr als 0,5 g/l und besonders bevorzugt von nicht mehr als 0,25 g/l.

Das anorganische Salz weist bevorzugt mindestens ein Anion auf, welches (PO₄³⁻), bedeutet.

Unter Phosphat sind auch die Kondensationsprodukte zu verstehen, wie beispielsweise Diphosphate, Triphosphate und Polyphosphate.

Das anorganische Salz weist bevorzugt mindestens ein Kation auf ausgewählt aus der Gruppe bestehend aus Alkalimetallen, Erdalkalimetallen, Ammonium, Cer, Eisen, Mangan, Chrom, Molybdän, Kobalt, Nickel oder Zink.

Bevorzugt sind Alkalimetalle und besonders bevorzugt sind Lithium, Natrium oder Kalium.

Besonders bevorzugt anorganische Salze sind Li₃PO₄, K₃PO₄, Na₃PO₄, sowie deren Hydrate, ganz besonders bevorzugt ist K₃PO₄.

K₃PO₄ kann erfindungsgemäß in wasserfreier Form eingesetzt werden sowie als Tri-, Hepta- oder Nonahydrat.

Die durch ein anorganisches Salz katalysierte Ver- oder Umesterung erfolgt im Allgemeinen bei 50 bis 140°C, bevorzugt bei 50 bis 100 °C, besonders bevorzugt bei 60 bis 90°C und ganz besonders bevorzugt bei 60 bis 80 °C.

Gegebenenfalls kann die Reaktion unter leichtem Vakuum von beispielsweise 300 hPa bis Normaldruck durchgeführt werden, wenn das bei der Veresterung freigesetzte Wasser oder der bei der Umesterung entstehende niedrigsiedende Alkohol, gegebenenfalls als Azeotrop, abdestilliert werden soll.

Das molare Verhältnis zwischen (Meth)Acrylsäure oder (Meth)Acrylsäureester und Alkohol (C) oder (D) beträgt bei der durch ein anorganisches Salz katalysierten Ver-oder Umesterung in der Regel 2 - 6 : 1 mol/mol, bevorzugt 2 - 3,5 : 1 mol/mol und besonders bevorzugt 2,5 - 3,0 : 1 mol/mol.

Die Reaktionszeit bei der durch ein anorganisches Salz katalysierten Ver- oder Umesterung beträgt in der Regel 30 min bis 24 Stunden, bevorzugt 45 min bis 18 Stunden, besonders bevorzugt 3 bis 12 Stunden und ganz besonders bevorzugt 5 bis 10 Stunden.

Der Gehalt an anorganischen Salzen im Reaktionsmedium liegt in der Regel im Bereich von etwa 0,01 bis 5 mol%, bevorzugt 0,1 -1,8 und besonders bevorzugt 0,3 - 1,5 mol% bezogen auf die Summe der eingesetzten Komponenten (C) bzw. (O).

Bei der Ver- oder Umesterung sind Polymerisationsinhibitoren (s.u.) zwingend erforderlich.

Die Anwesenheit von sauerstoffhaltigen Gasen (s.u.) während der durch ein anorganisches Salz katalysierten Reaktion ist bevorzugt.

Bei der durch ein anorganisches Salz katalysierten Ver- oder Umesterung werden in der Regel die Produkte mit einer Farbzahl unter 500 APHA, bevorzugt unter 200 und besonders bevorzugt unter 150 (gemäß DIN ISO 6271) erhalten.

Erfindunqsaemäß einsetzbare Enzvme (E) sind beispielsweise ausgewählt aus der Gruppe bestehend aus Lipase aus Candida antarctica B, Lipase aus Alcaligenes sp., Aspergillus sp., Mucor sp., Penicilium sp., Geotricum sp., Rhizopus sp., Burkholderia sp., Candida sp., Pseudomonas sp., Thermomyces sp. oder Schweinepankreas.

Die enzymatische Ver- oder Umesterung mit einem (Meth)acrylat erfolgt im Allgemeinen bei 20 bis 100°C, bevorzugt 20 bis 80°C, besonders bevorzugt 30 bis 70°C, ganz besonders bevorzugt 40 bis 60 °C.

Die Obergrenze der Temperatur ist dabei natürlich der Siedepunkt des Lösungsmittels oder des bei einer Umesterung freigesetzten Alkohols.

Gegebenenfalls kann die Reaktion unter leichtem Vakuum von beispielsweise 300 hPa bis Normaldruck durchgeführt werden, wenn das bei der Veresterung freigesetzte Wasser oder der bei der Umesterung entstehende niedrigsiedende Alkohol, gegebenenfalls als Azeotrop, abdestilliert werden soll.

Der Enzymgehalt im Reaktionsmedium liegt in der Regel im Bereich von etwa 0,1 bis 10 Gew.-%, bezogen auf die Summe der eingesetzten Komponenten (C) bzw. (O) und (D).

Das molare Verhältnis zwischen (Meth)Acrylsäure oder (Meth)Acrylsäureester und Alkohol (C) oder (D) beträgt bei der enzymatisch katalysierten Ver- oder Umesterung in der Regel 1 - 50 : 1 mol/mol, bevorzugt 5 - 20 : 1 mol/mol.

Die Reaktionszeit bei der enzymatisch katalysierten Ver- oder Umesterung beträgt in der Regel 6 Stunden bis 3 Tage, bevorzugt 8 Stunden bis 2 Tage und besonders bevorzugt 12 bis 24 Stunden.

Bevorzugt wird die Reaktionszeit so angepaßt, daß der Umsatz aller im Alkohol enthaltenden Hydroxyfünktionen mindestens 70%, bevorzugt mindestens 80, besonders bevorzugt mindestens 90, ganz besonders bevorzugt mindestens 95 % und insbesondere mindestens 97 % beträgt.

Die enzymatisch katalysierte Reaktion kann optional auch in Abwesenheit von Stabilisatoren (s.u.) ausgeführt werden, bevorzugt wird sie in Gegenwart mindestens eines Stabilisators ausgeführt.

Die bei der enzymatisch katalysierten Reaktion erhältlichen Reaktionsprodukte weisen in der Regel eine Farbzahl von weniger als 100 APHA, bevorzugt unter 80 APHA auf.

Die folgenden Reaktionsparameter gelten, wenn nicht anders angegeben, sowohl für die enzymatisch als auch die mit anorganischen Salzen katalysierte Reaktion.

Die Reaktion kann in organischen Lösungsmitteln oder deren Gemischen oder ohne Zusatz von Lösungsmitteln ablaufen. Die Ansätze sind in der Regel weitgehend wasserfrei (d.h. unter 10, bevorzugt unter 5, besonders bevorzugt unter 1 und ganz besonders bevorzugt unter 0,5 Gew% Wassergehalt). Ferner sind die Ansätze weitgehend frei von primären und sekundären Alkoholen, d.h. unter 10, bevorzugt unter 5, besonders bevorzugt unter 1 und ganz besonders bevorzugt unter 0,5 Gew% Alkoholgehalt.

Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxyethan, Diethylenglycoldimethylether, Polyethylenglycoldimethylether 500, C₁-C₄-Alkylencarbonate, insbesondere Propylencarbonat, Essigsäure-C₃-C₆-alkylester, insbesondere Essigsäure-tert.-butylester, THF, Toluol, 1,3-Dioxolan, Aceton, iso-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen.

In einer besonders bevorzugten Ausführungsform der Umesterung wird die Reaktion in dem als Edukt eingesetzten (Meth)Acrylsäureester durchgeführt. Ganz besonders bevorzugt ist die Durchführung der Reaktion in der Weise, daß das Produkt (C) oder (O) nach Beendigung der Reaktion als etwa 10 - 80 Gew%ige Lösung in dem als Edukt eingesetzten (Meth)Acrylsäureester anfällt, insbesondere als 20 bis 50 Gew%ige Lösung.

Die Edukte liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l, insbesondere bei 0,15 bis 10 Mol/l oder 0,2 bis 5 mol/l liegt.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Die Durchmischung kann beispielsweise durch Einspeisen eines Gases, bevorzugt eines sauerstoffhaltigen Gases (siehe unten) erfolgen. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlauf erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum der freigesetzte Alkohol gleichzeitig abdestilliert werden kann.

Die Entfernung von Wasser im Falle einer Veresterung oder Alkoholen, die bei einer Umesterung aus den Alkyl(meth)acrylaten freigesetzt werden, erfolgt kontinuierlich oder schrittweise in an sich bekannter Weise, z.B. durch Vakuum, azeotrope Entfernung, Strippen, Absorption, Pervaporation und Diffusion über Membranen oder Extraktion.

Das Strippen kann beispielsweise durch Durchleiten eines sauerstoffhaltigen Gases, bevorzugt eines Luft oder Luft-Stickstoff-Gemisches, durch das Reaktionsgemisch erfolgen, gegebenenfalls zusätzlich zu einer Destillation.

Zur Absorption eignen sich vorzugsweise Molekularsiebe oder Zeolithe (Porengröße z.B. im Bereich von etwa 3-10 Angström), eine Abtrennung durch Destillation oder mit Hilfe geeigneter semipermeabler Membranen.

Es ist aber auch möglich, das abgetrennte Gemisch aus Alkyl(meth)acrylat und dem diesem zugrundeliegenden Alkohol, das häufig ein Azeotrop bildet, direkt in eine Anlage zur Herstellung des Alkyl(meth)acrylats zuzuführen, um es dort in einer Veresterung mit (Meth)acrylsäure wiederzuverwerten.

Es kann bei der enzymatisch katalysierten Reaktion vorteilhaft sein, freiwerdendes Wasser oder Alkohol durch ein möglichst nahe am Temperaturoptimum des verwendeten Enzyms siedendes binäres oder ternäres Heteroazeotrop abzutrennen. Der so entfernte Alkohol kann dann durch Phasenscheidung oder Membrandampftrennung entfernt werden.

Nach Beendigung der Reaktion kann man das aus c) erhältliche Reaktionsgemisch ohne weitere Aufreinigung weiterverwenden oder es erforderlichenfalls in einem weiteren Schritt d) aufreinigen.

In der Regel wird im Schritt d) lediglich der eingesetzte heterogene Katalysator vom Reaktionsgemisch abgetrennt und das Reaktionsprodukt vom gegebenenfalls verwendeten organischen Lösungsmittel abgetrennt.

Eine Abtrennung vom heterogenen Katalysator erfolgt in der Regel durch Filtration, Elektrofiltration, Absorption, Zentrifugation oder Dekantieren. Der abgetrennte heterogene Katalysator kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie durchgeführt werden.

Bevorzugt werden in Schritt d) jedoch lediglich der heterogene Katalysator und das gegebenenfalls eingesetzte Lösungsmittel abgetrennt.

Die Reaktionsbedingungen bei der erfindungsgemäßen, besonders bei der enzymatischen Ver- oder Umesterung sind mild. Aufgrund der niedrigen Temperaturen und sonstigen milden Bedingungen wird die Bildung von Nebenprodukten in Schritt c) vermieden, die andernfalls zum Beispiel von chemischen Katalysatoren stammen können oder durch unerwünschte radikalische Polymerisation des eingesetzten (Meth)acrylats, die sonst nur durch Zugabe von Stabilisatoren verhindert werden kann. Bei der erfindungsgemäßen Reaktionsführung kann der (Meth)acrylverbindung (D) über den ohnehin enthaltenen Lagerstabilisator hinaus zusätzliche Stabilisator in mindestens das eingesetzte (Meth)acrylat oder in die Reaktionsmischung zugegeben werden, beispielsweise Hydrochinonmonomethylether, Phenothiazin, Phenole, wie z.B. 2-tert.-Butyl-4-methylphenol, 6-tert.-Butyl-2,4-dimethyl-phenol oder N-Oxyle, wie 4-Hydroxy-2,2,6,6-tetramethyl-piperidin-N-oxyl, 4-Oxo-2,2,6,6-tetramethyl-piperidin-N-oxyl oder Uvinul® 4040P der BASF Aktiengesellschaft, beispielsweise in Mengen von 50 bis 2000 ppm.

Vorteilhaft wird die Ver- oder Umesterung in Gegenwart eines sauerstoffhaltigen Gases, bevorzugt Luft oder Luft-Stickstoff-Gemische, durchgeführt.

Der erfindungsgemäß verwendete heterogene Katalysator kann unproblematisch vom Endprodukt entfernt werden, durch die erfindungsgemäß geringe Löslichkeit des heterogenen Katalysators im Reaktionsmedium verbleiben höchstens unwesentliche Spuren des Katalysators im Produkt. Dadurch reicht zur Aufarbeitung des Reaktionsproduktes in der Regel eine einfache Filtration oder Dekantierung, auf aufwendige Wäschen, Neutralisationen und dergleichen zur Abtrennung des Katalysators kann durch das erfindungsgemäße Verfahren verzichtet werden.

Die erfindungsgemäß eingesetzten Katalysatoren zeigen eine geringe Tendenz zu Nebenreaktionen. Die anorganischen Salze sind ausreichend basisch um eine Ver- oder Umesterung zu katalysieren aber nicht zu basisch, um Nebenreaktionen wie beispielsweise die eingangs erwähnte Michael-Reaktion zu katalysieren, so daß durch das erfindungsgemäße Verfahren der Anteil von Michael-Addukten gegenüber aus dem Stand der Technik bekannten Reaktionen deutlich verringert werden kann.

Des weiteren ist die Reaktion unter den erfindungsgemäßen Reaktionsbedingungen sehr selektiv, man findet in der Regel weniger als 10 %, bevorzugt weniger als 5 % Nebenprodukte (bezogen auf den Umsatz).

Typische Nebenprodukte der Umsetzung der Verbindungen (C) und (O), in denen Z¹ N-H ist, mit (Meth)Acrylsäure oder (Meth)Acrylsäureestern sind das über dieses Stickstoffatom verknüpfte (Meth)Acrylamid, das über dieses Stickstoffatom verknüpfte Michael-Addukt, das über die freie Hydroxygruppe verknüpfte Michael-Addukt, sowie Produkte, die aus einer intra- oder intermolekularen Abspaltung der Gruppe R⁵-Z¹ aus den Verbindungen (C) oder (O) resultieren, die dann wiederum ver- oder umgeestert werden können.

Ein besonderen Vorteil der erfindungsgemäßen Katalysatoren ist, daß der Anteil von mehrfach, also mindestens zweifach (meth)acrylierten Produkten zurückgedrängt werden konnte. Derartige Produkte sind aus dem mono(meth)acrylierten Wunschprodukt nur schwer abtrennbar. Da das Wunschprodukt als Monomer oder Reaktivverdünner eingesetzt werden soll (siehe unten) ist ein Gehalt an mehrfach (meth)acryliertem Produkt unerwünscht, da derartige Produkte zu Vernetzungen führen, die in Polymerisationen das Molekulargewicht der Produkte unvorhersagbar erhöhen können.

Die erfindungsgemäß hergestellten (Meth)acrylate von N-hydroxyalkylierten Amiden finden Anwendung beispielsweise als Monomere oder Comonomere in der Herstellung von Dispersionen, beispielsweise Acryldispersionen, als Reaktivverdünner, beispielsweise in strahlungshärtbaren Beschichtungsmassen oder in Farben, bevorzugt in Außenfarben, sowie in Dispersionen für Anwendung im Papierbereich.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

Die Apparatur bestand aus einem 500mL-Vierhalsrundkolben mit Lufteinleitungsrohr, Trennkolonne, Probenahmestelle und Innentemperaturmessfühler. Gerührt wurde mit einem Magnetrührer bei 300 rpm.

Als Trennkolonne kam eine 20 cm Vigreuxkolonne mit Raschigringen (5x5 mm) zum Einsatz. Auf der Trennkolonne wurde ein Kolonnenkopf mit Rückflussregler und Liebigkühler aufgesetzt und konnte in allen Experimenten ohne Ablagerungen von Polymer betrieben werden.

Die einzelnen Ansätze wurden nach folgender allgemeiner Arbeitsvorschrift durchgeführt: 2-Hydroxyethyl ethylenharnstoff (HEEH, 130 g, 1,0 Mol) wurde aufgeschmolzen und mit der entsprechenden Menge an Methylmethacrylat (MMA) überschichtet (5,0 Mol, 560 g). Beim Hochheizen auf 100°C Innentemperatur wurde der Katalysator (0,8 mol-% bezogen auf den Harnstoff) zugegeben. Das entstehende Azeotrop aus Methylmethacrylat (MMA) und Methanol wurde bei einem Rückflussverhältnis von 2:1 abdestilliert (65°C). In regelmäßigen Abständen wurden Proben von Destillat und Sumpf gezogen und analysiert. Nach dem Ende der Methanolentwicklung wurde noch mit einem Rücklaufverhältnis von 10:1 gefahren, bis sechs Stunden Versuchsdauer erreicht waren. Dann wurde die Destillation abgestellt und der Sumpf gaschromatographisch auf das Produkt Methacrylsäure 2-(2-oxo-imidazolidin-1-yl)-ethyl ester sowie entstandene Nebenkomponenten (in Summe) untersucht. Die aufgefundenen Nebenkomponenten sind u.a. N-[2-(2-oxo-oxazolidin-3-yl)-ethyl]-methacrylamid, N-{2-[3-(Methacryloyl)-2-oxo-imidazolidin-1-yl]-ethyl}-methacrylamid, 2-Methyl-3-{3-[2-(methacryloylamino)-ethyl]-2-oxo-imidazolidin-1-yl}-propionsäure und 2-Methyl-3-[2-(2-oxo-imidazolidin-1-yl)-ethoxy]-propionsäure.

### Beispiel 1: Kaliumphosphat

| Katalysator (mol-%) | MMA [FI.-%] | HEEH [FI.-%] | Produkt [FI.-%] | Dimethacrylat [FI.-%] |
|---|---|---|---|---|
| Kaliumphosphat (1,5) | 79,05 | 2,24 | 16,38 | 0,31 |
| Kaliumphosphat (1,0) | 77,55 | 2,34 | 17,93 | 0,32 |
| Kaliumphosphat (0,5) | 73,55 | 2,42 | 20,21 | 0,57 |

### Vergleichsbeispiel 1: Kaliumcarbonat

| Katalysator (mol-%) | MMA [FI.-%] | HEEH [FI.-%] | Produkt [FI.-%] | Dimethacrylat [FI.-%] |
|---|---|---|---|---|
| Kaliumcarbonat (0,2) | 78,65 | 1,38 | 16,16 | 0,48 |

### Vergleichsbeispiel 2: Dibutylzinnoxid (DBTO)

| Katalysator (mol-%) | MMA [FI.-%] | HEEH [FI.-%] | Produkt [FI.-%] | Dimethacrylat [FI.-%] |
|---|---|---|---|---|
| DBTO (0,8) | 71,21 | 7,2 | 20,13 | 1,07 |

### Vergleichsbeispiel 3: Kaliummethylat

| Katalysator | MMA [FI.-%] | HEEH [FI.-%] | Produkt [FI.-%] | Dimethacrylat [FI.-%] |
|---|---|---|---|---|
| K-methylat (1 mol%) | 79,17 | 8,24 | 9,97 | 0,11 |

Der Vergleich der Versuche zeigt eindeutig, dass Kaliumphosphat als Katalysator den bestehenden Systemen überlegen ist. Bei mindestens verdoppelter Raum-ZeitAusbeute und geringem Anteil an Vernetzer verläuft die Umesterung mit hoher Selektivität und hohem Umsatz. Der Katalysator fällt beim Abkühlen in Form von gut filtrierbaren Kristallen aus und ein Waschschritt entfällt.

### Beispiel 3: Enzymkatalyse

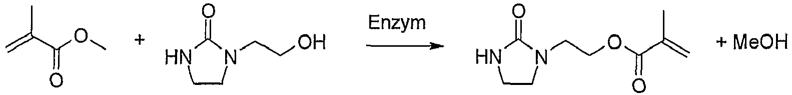

10 mmol 2-Hydroxyethyl ethylenharnstoff (HEEH, 1,3 g), 100 bzw. 400 mmol Methylmethacrylat, 2,0 g Molekularsieb (5 Å) und 65-260 mg Novozym® 435 (immobilisierte Lipase aus Candida antartica B, Fa. Novozymes) wurden 72 h bei 40 °C in einem Schraubdeckelglas geschüttelt. Man erhielt farblose Lösungen.

Zur Analysenvorbereitung wurde eine homogene Probe hergestellt, indem eventuelle Niederschläge durch Zugabe von 10 ml 1,4-Dioxan aufgelöst wurden. Der Umsatz von HEEH zum Methacrylat wurde mittels der obigen GC-Methode gaschromatographisch bestimmt.

Ansätze mit 100 mmol MMA

| eingesetzte Enzymmenge | HEEH [FI.-%) | Produkt [FI.-%] | ∑ Nebenkomp [FI.-%] | Umsatz [%] |
|---|---|---|---|---|
| 130 mg | 46,1 | 52,9 | 1,0 | 53 |
| 260 mg | 33,3 | 65,8 | 0,8 | 66 |

Ansätze mit 400 mmol MMA

| eingesetzte Enzymmenge | HEEH [FI.-%] | Produkt [FI.-%] | ∑ Nebenkomp [FI.-%] | Umsatz [%] |
|---|---|---|---|---|
| 65 mg | 41,7 | 57,1 | 0,8 | 58 |
| 130 mg | 43,4 | 55,7 | 0,7 | 56 |
| 260 mg | 13,2 | 86,1 | 0,6 | 87 |

## Patentansprüche

1. Verfahren zur Herstellung von (Meth)acrylaten von N-hydroxyalkylierte Amide in dem man ringförmige N-hydroxyalkylierte Amide (C), 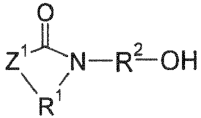 oder offenkettige N-hydroxyalkylierte Amide (O) 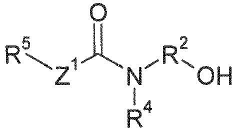 worin
Z¹ Sauerstoff, Schwefel, unsubstituierter oder substituierter Phosphor oder mono-oder unsubstituierter Stickstoff (N-R³),
R¹ und R² jeweils unabhängig voneinander C₂-C₂₀-Alkylen, C₅-C₁₂-Cycloakylen, C₆-C₁₂-Arylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, oder
R²-OH für eine Gruppe der Formel -[Xᵢ]ₖ-H,
R³, R⁴ und R⁵ unabhängig voneinander jeweils Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünfbis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, wobei R³ und R⁵ auch gemeinsam einen fünf- bis zwölfgliedrigen Ring bilden können,
mit der Maßgabe, daß im Fall von Z¹ = O R⁵ lediglich unsubstituiertes C₁ - C₁₈-Alkyl, C₆ - C₁₂-Aryl oder C₅ - C₁₂-Cycloalkyl ist,
k für eine Zahl von 1 bis 50 und
Xᵢ für jedes i = 1 bis k unabhängig voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O- und -CHPh-CH₂-O-, worin Ph für Phenyl und Vin für Vinyl steht,
bedeuten,
in Gegenwart mindestens eines heterogenen Katalysators ausgewählt aus der Gruppe bestehend aus anorganischen Phosphat-Salzen (S), wobei das mindestens eine anorganische Phosphat-Salz (S) einen pK_{B}-Wert von nicht mehr als 7,0 und nicht weniger als 1,0 sowie einen Löslichkeit im Reaktionsmedium bei 25°C von nicht mehr als 1 g/l aufweist, und Enzymen (E), wobei das Enzym ausgewählt ist aus der Gruppe bestehend aus Lipase aus Candida antarctica B, Lipase aus Alcaligenes sp., Aspergillus sp., Mucor sp., Penicilium sp., Geotricum sp., Rhizopus sp., Burkholderia sp., Candida sp., Pseudomonas sp., Thermomyces sp. oder Schweinepankreas, mit (Meth)acrylsäure verestert oder mindestens einem (Meth)acrylsäureester (D) umestert.

2. Verfahren gemäß Anspruch 1, **dadurch gekennzeichnet, daß** Z¹ un- oder monosubstituierter Stickstoff (N-R³) ist.

3. Verfahren gemäß einem der vostehenen Ansprüche, **dadurch gekennzeichnet, daß** das anorganische Phosphat-Salz mindestens ein Kation aufweist, ausgewählt aus der Gruppe bestehend aus Alkalimetallen, Erdalkalimetallen, Ammonium, Cer, Eisen, Mangan, Chrom, Molybdän, Kobalt, Nickel oder Zink.

4. Verfahren gemäß einem der vostehenen Ansprüche, **dadurch gekennzeichnet, daß** das anorganische Phosphat-Salz ausgewählt ist aus der Gruppe bestehend aus Li₃PO₄, K₃PO₄, Na₃PO₄, sowie deren Hydrate.

5. Verfahren nach einem der vorstehenden Ansprüche, **dadurch gekennzeichnet, daß** man die Reaktion in der Weise durchführt, daß das Produkt (C) oder (O) nach Beendigung der Reaktion als etwa 10 - 80 Gew%ige Lösung in dem als Edukt eingesetzten (Meth)Acrylsäureester anfällt.

## Claims

1. A process for preparing (meth)acrylates of N-hydroxyalkylated amides, in which cyclic N-hydroxyalkylated amides (C) 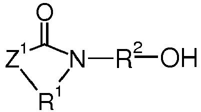 or open-chain N-hydroxyalkylated amides (0) 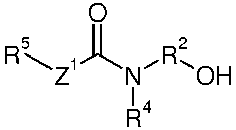 where
Z¹ is oxygen, sulfur, unsubstituted or substituted phosphorus or mono- or unsubstituted nitrogen (N-R³),
R¹ and R² are each independently C₂-C₂₀-alkylene, C₅-C₁₂-cycloalkylene, C₆-C₁₂-arylene, or C₂-C₂₀-alkylene interrupted by one or more oxygen and/or sulfur atoms and/or one or more substituted or unsubstituted imino groups and/or by one or more cycloalkyl, -(CO)-,-O(CO)O-, -(NH)(CO)O-, -O(CO)(NH)-, -O(CO)- or -(CO)O-groups, where the radicals mentioned may each be substituted by aryl, alkyl, aryloxy, alkyloxy, heteroatoms and/or heterocycles, or
R²-OH is a group of the formula -[Xᵢ]ₖ-H,
R³, R⁴ and R⁵ are each independently hydrogen, C₁-C₁₈-alkyl, or C₂ - C₁₈-alkyl, C₂ - C₁₈-alkenyl, C₆-C₁₂-aryl, C₅ - C₁₂-cycloalkyl which are each optionally interrupted by one or more oxygen and/or sulfur atoms and/or one or more substituted or unsubstituted imino groups, or a five- to six-membered heterocycle having oxygen, nitrogen and/or sulfur atoms, where the radicals mentioned may each be substituted by aryl, alkyl, aryloxy, alkyloxy, heteroatoms and/or heterocycles, and R³ and R⁵ may also together form a five- to twelve-membered ring,
with the proviso that, in the case that Z¹ = 0, R⁵ is only unsubstituted C₁ - C₁₈-alkyl, C₆ - C₁₂-aryl or C₅ -C₁₂-cycloalkyl,
kis a number from 1 to 50 and
Xᵢ for each i = 1 to k may independently be selected from the group of -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- and -CHPh-CH₂-O- where Ph is phenyl and Vin is vinyl,
are esterified with (meth) acrylic acid or transesterified with at least one (meth)acrylic ester (D) in the presence of at least one heterogeneous catalyst selected from the group consisting of inorganic phosphate salts (S), wherein the at least one inorganic phosphate salt (S) has a pK_{B} of not more than 7.0 and not less than 1.0, and a solubility in the reaction medium at 25°C of not more than 1 g/l, and enzymes (E), wherein the enzyme is selected from the group consisting of lipase from Candida antarctica B, lipase from Alcaligenes sp., Aspergillus sp., Mucor sp., Penicilium sp., Geotricum sp., Rhizopus sp., Burkholderia sp., Candida sp., Pseudomonas sp., Thermomyces sp. or porcine pancreas.

2. The process according to claim 1, wherein Z¹ is unsubstituted or monosubstituted nitrogen (N-R³).

3. The process according to either of the preceding claims, wherein the inorganic phosphate salt has at least one cation selected from the group consisting of alkali metals, alkaline earth metals, ammonium, cerium, iron, manganese, chromium, molybdenum, cobalt, nickel and zinc.

4. The process according to any of the preceding claims, wherein the inorganic phosphate salt is selected from the group consisting of Li₃PO₄, K₃PO₄, Na₃PO₄ and hydrates thereof.

5. The process according to any of the preceding claims, wherein the reaction is carried out in such a way that the product (C) or (0) is obtained on completion of the reaction as an about 10 - 80% by weight solution in the (meth)acrylic ester used as the reactant.

## Revendications

1. Procédé pour la préparation de (méth)acrylates d'amides N-hydroxyalkylés, dans lequel des amides cycliques, N-hydroxyalkylés (C), 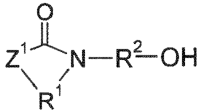 ou des amides N-hydroxyalkylés à chaîne ouverte (0) 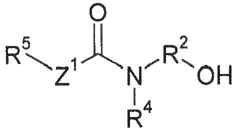 dans laquelle
Z¹ signifie oxygène, soufre, phosphore non substitué ou substitué ou azote monosubstitué ou non substitué (N-R³) ,
R¹ et R² signifient, indépendamment, C₂-C₂₀-alkylène, C₅-C₁₂-cycloalkylène, C₆-C₁₂-arylène; ou C₂-C₂₀-alkylène interrompu par un ou plusieurs atomes d'oxygène et/ou de soufre et/ou par un ou plusieurs groupes imino substitués ou non substitués et/ou par un ou plusieurs groupes cycloalkyle, - (CO) -, -O(CO)O-, -(NH)(CO)O-, -O(CO)(NH)-, -O(CO)- ou -(CO)O-, les radicaux mentionnés pouvant être à chaque fois substitués par aryle, alkyle, aryloxy, alkyloxy, des hétéroatomes et/ou des hétérocycles ou R²-OH signifie un groupe de formule -[Xᵢ]ₖ-H,
R³, R⁴ et R⁵ signifient, indépendamment les uns des autres, hydrogène, C₁-C₁₈-alkyle ; C₂-C₁₈-alkyle, C₂-C₁₈-alcényle, C₆-C₁₂-aryle, C₅-C₁₂-cycloalkyle le cas échéant interrompu par un ou plusieurs atomes d'oxygène et/ou de soufre et/ou un ou plusieurs groupes imino substitués ou non substitués ; ou un hétérocycle de cinq à six chaînons, présentant des atomes d'oxygène, d'azote et/ou de soufre, les radicaux mentionnés pouvant à chaque fois être substitués par aryle, alkyle, aryloxy, alkyloxy, des hétéroatomes et/ou des hétérocycles, R³ et R⁵ pouvant également former ensemble un cycle de cinq à douze chaînons, à condition que, dans le cas où Z¹ = 0, R⁵ représente uniquement C₁-C₁₈-alkyle, C₆-C₁₂-aryle ou C₅-C₁₂-cycloalkyle non substitué,
k vaut un nombre de 1 à 50 et
Xᵢ pour chaque i = 1 à k, peut être choisi, indépendamment, dans le groupe formé par -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(OH₃)-O-, CH(CH₃)-CH₂-O-, -CH₂-C(-CH₃)₂-O-, -C(CH₃)₂-CH₂-O-, -CH₂-CH₂-CH₂-O-, -CH₂-CH₂-CH₂-CH₂-O-, -CH₂-CHVin-O-, CHVin-CH₂-O-, -CH₂-CHPh-O- et -CHPh-OH₂-O-, où Ph représente phényle et Vin représente vinyle,
en présence d'au moins un catalyseur hétérogène, choisi dans le groupe constitué par les sels de phosphate inorganiques (S), ledit au moins un sel de phosphate inorganique (S) présentant une valeur pK_{B} qui n'est pas supérieure à 7,0 et pas inférieure à 1,0 ainsi qu'une solubilité dans le mélange réactionnel à 25°C qui n'est pas supérieure à 1 g/l, et d'enzymes (E), l'enzyme étant choisie dans le groupe constitué par la lipase de Candida antarctica B, la lipase d'Alcaligenes sp., d'Aspergillus sp., de Mucor sp., de Penicilium sp., de Geotricum sp., de Rhizopus sp., de Burkholderia sp., de Candida sp., de Pseudomonas sp., de Thermomyces sp. ou de pancréas de porc, sont estérifiés avec de l'acide (méth)acrylique ou transestérifiés avec au moins un ester de l'acide (méth) acrylique (D).

2. Procédé selon la revendication 1, **caractérisé en ce que** Z¹ représente un azote non substitué ou monosubstitué (N-R³) .

3. Procédé selon l'une quelconque des revendications précédentes, **caractérisé en ce que** le sel de phosphate inorganique présente au moins un cation choisi dans le groupe constitué par les métaux alcalins, les métaux alcalino-terreux, l'ammonium, le cérium, le fer, le manganèse, le chrome, le molybdène, le cobalt, le nickel ou le zinc.

4. Procédé selon l'une quelconque des revendications précédentes, **caractérisé en ce que** le sel de phosphate inorganique est choisi dans le groupe constitué par Li₃PO₄, K₃PO₄, Na₃PO₄ ainsi que leurs hydrates.

5. Procédé selon l'une quelconque des revendications précédentes, **caractérisé en ce que** la réaction est réalisée de manière telle que le produit (C) ou (0), après la fin de la réaction, est obtenu sous forme d'une solution à environ 10-80% en poids dans l'ester de l'acide (méth)acrylique utilisé comme produit de départ.
